# NEW EUROPEAN PATENT SPECIFICATION

(11) **EP 3 032 975 B2**
(45) Date of publication and mention of the opposition decision: **24.04.2024**
(45) Mention of the grant of the patent: 29.05.2019
(21) Application number: 14836345.0
(22) Date of filing: 14.08.2014
(51) Int. Cl.: A24F 47/00, A61M 15/06, H05B 3/80, A61M 11/04, A24B 15/16

(54) **APPARATUS AND METHOD FOR CONTROLLING ELECTRIC VAPORIZER**
VORRICHTUNG UND VERFAHREN ZUR STEUERUNG EINES ELEKTRISCHEN ZERSTÄUBERS
APPAREIL ET PROCÉDÉ DE COMMANDE D'UN VAPORISATEUR ÉLECTRIQUE

(30) Priority: 14.08.2013 FI 20135829
(43) Date of publication of application: 22.06.2016
(73) Proprietor: Pixan OY, 90460 Oulunsalo (FI)
(72) Inventor: KANANEN, Mika, FI-90460 Oulunsalo (FI)
(74) Representative: Berggren Oy
(86) International application number: PCT/FI2014/050624
(87) International publication number: WO 2015/022448

(56) References cited:
- EP-A1- 2 110 033
- EP-A1- 2 110 033
- EP-A1- 2 399 636
- EP-A1- 2 399 636
- WO-A1-2013/060781
- WO-A2-2013/098398
- US-A- 6 040 560
- US-A1- 2003 226 837
- US-A1- 2010 024 816
- US-A1- 2011 097 060
- US-A1- 2011 226 236
- US-A1- 2012 174 914
- US-A1- 2012 174 914
- US-A1- 2013 104 916
- US-A1- 2013 104 916

## Description

### Field of the invention

The invention relates generally to electronic vaporizers. Specifically the invention relates to controlling electric vaporizers.

### Background of the invention

The following description of background art may include insights, discoveries, understandings or disclosures, or associations together with disclosures not known to the relevant art prior to the present invention but provided by the invention. Some such contributions of the invention may be specifically pointed out below, whereas other such contributions of the invention will be apparent from their context.

In recent year electronic vaporizers have been developed. One use for the vaporizers is to simulate smoking. The electronic vaporizers comprise a heating element configured to vaporize given material, typically liquid material, which is then inhaled by the user. The vaporizers comprise a power source for the heating element and some sort of controlling element for the vaporizing process.

The usage experience of electronic vaporizers depends on the components and the control process of the components of the vaporizer. The choice of the material to be inhaled is naturally important for the usage experience. Liquids with different flavours lead to different results. In addition, different types of heating elements and different power fed to the heating element have a strong effect on the usage experience. It has been noticed that best results are achieved when the power fed to the heating element is as constant as possible.

Reference WO 2013/098398 ("Philip Morris / Talon") discusses an aerosol generating system with consumption monitoring and feedback. Thus, in other words, it gives feedback to the e-cigarette user e.g. through showing composition of different substances of the liquid on the screen. It seems that Talon is able to e.g. show personal consumption amounts of nicotine and if a certain threshold value is exceeded in a time period, a warning can be given to the user. Changes in the air flow past the heating element can be measured or detected. Starting from p. 17 I. 3 and especially in p. 17 I. 31-35 it is mentioned that voltage "V" and current "I" are measured, and thus the heating element resistance "R" is obtained, and after that, the resistivity is obtained as well. Temperature/resistivity pairs of values can be gathered in a look-up table and based on the resistance measurement, the temperature of the heating element can be determined. In order to activate the power feeding to the heating element, the user must have been activated the vaporizer and also the liquid to be heated must exist around the heating element (this feature has an own detector "32"), see the end of page 18. If the desired target temperature is higher than the present true temperature of the element obtained by the resistance measurement, the controller increases power to the element, and vice versa in an opposite situation. Furthermore, the effect of air flow based cooling on the heating element is taken into account as affecting the temperature of the heating element. Regarding p. 23 I. 12-21 in Talon, the referred normalization of the power values, it discusses comparing the power values against different threshold values for distinguishing different liquid materials. Also it is possible to detect an unappropriate liquid material or the finished, i.e. fully consumed, liquid in the compartment.

Reference WO 2013/060781 ("Philip Morris / Flick") discloses an aerosol generating system with improved aerosol production. Flick measures temperature of the heating element, and the controller adjusts the power fed to the heating element to maintain the temperature of the heating element within a desired temperature range. Furthermore, the desired temperature is dynamically calculated based on measured air flow. Thus, the inhaling action of the user defines how much power is fed to the heating element. This principle gives a more consistent type of aerosol independent of the variations in the (subsequent) inhalations of the user.

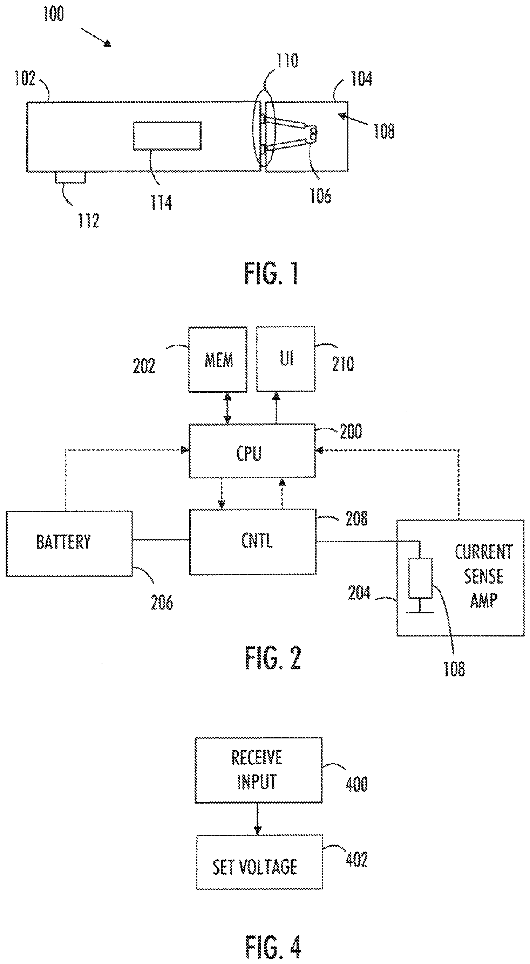

Flick uses voltage division for determining an additional resistor value "R3" within the insertable liquid compartment, while resistance "R2" is part of the e-cigarette device itself. According to page 16 lines 19-27 of Flick, a look-up table comprises resistance values of "R3", and corresponding temperature ranges or resistance ranges for the heating element "119" (see Fig. 4). As a result, the heating element will not heat into too high temperature, when the device operates within those ranges. In Flick it is possible to compare just the resistances, and thus, a separate temperature sensor is not required.

Reference US 2010/0024816 ("Weinstein") describes an inline vaporizer, which adds vaporized fluid to actual carrier gas line, resulting in a mixture of gases. The application area of Weinstein is medical technology where e.g. vaporized substance, such as medicine or plain water vapour, can be added to the air inhaled by the patient. In determining the temperature of the heating element, calculation logic according to paragraphs [0120]-[0121] is applied, and thus, the voltage and current over a sense resistor "Rs" are measured. There is a data table where resistor and temperature values are linked together. Weinstein applies a relatively complex calculation logic for calculating temperature of the heating material based in the resistance comparison, and based on impedance changes as a function of temperature changes. According to par. [0018], the system may have "a safety controller" which observes that acceptable operational parameters are maintained all the time. Such a physical attribute of the system can be an operating power of the system or the temperature of the system. Weinstein thus parallels these two quantities.

Furthermore in Weinstein, the flow rate of the pump can be measured with a sensor, the measured flow rate can be converted with an ADC, and the resulting value can be compared to a value picked from the data table. As another option, the temperature or the power consumption of the heating element can be observed, and this can be performed e.g. by a thermocouple (par. [0080]), and the AD-converted sensor data can be compared to a data table value, and thus, the breathing rhythm of the patient can be obtained (because inhaling and exhaling has an effect on the temperature of the heating element). Furthermore, the power consumption of the heating element can be controlled, referring at the same time to the desired vapour amount (volume). Generally, many types of sensor data can be AD-converted, and the obtained value can be compared to a data table value for finding out a desired physical quantity.

Reference US 2012/174914 ("Pirshafiey") discloses an electronic vapor inhaling device, i.e. an e-cigarette, incorporating various electrically powered control components that manage the operation of the device. The control components adjust the wattage to an atomizer by reading an RFID tag or barcode which is disposed on a cartridge comprising the liquid material. This kind of a cartridge ID is uniquely associated with a certain liquid in a particular cartridge. The cartridge ID is connected, i.e. linked, to the intrinsic resistance of the liquid. The processor receives the ID data when the cartridge is connected to the device, links the ID data with the resistance value picked from the memory and adjusts automatically the power fed to the atomizer based on the picked resistance value.

Reference EP 2399636 ("Philip Morris / Thorens") discloses an aerosol generating system and a corresponding liquid storage portion. Referring to Figure 7, a part of the measurement circuit is within the liquid and another part is outside the liquid in connection with the controller. There are either one or two resistors in parallel or series within the liquid. When the liquid cartridge is connected to the e-cigarette device, the circuit closes and it is noted that the liquid cartridge is indeed in its place and the heating element can be turned on. With a traditional voltage/current measurement the resistance of the resistor or the resistor circuit can be measured. The controller uses a look-up table so that the defined resistance value acts as a search parameter. The look-up table comprises resistance values paired with corresponding ID data of different liquid storage portions. Based on the measured resistance value, the closest resistance is picked from the look-up table, and thus, the corresponding liquid cartridge ID is achieved. The controller notices the change of the liquid compartment as well. Each resistance value in the look-up table can be linked with an "energy profile" parameter which describes the energy fed to the heating element. The purpose of this device is to produce a constant amount of vapour regardless of the type of liquid, or the type of the liquid storage portion, or regardless of any changes of the storage portions. In Thorens, the heating element is a different component than the resistors used in the measurement.

### Brief description

According to an aspect of the present invention, there is provided a controller of an electronic vaporizer, as specified in claim 1.

According to another aspect of the present invention, there is provided a method for controlling an electronic vaporizer, as specified in claim 7.

### List of drawings

Embodiments of the present invention are described below, by way of example only, with reference to the accompanying drawings, in which
Figure 1 illustrates an example of an electric vaporizer;
Figure 2 illustrates another example of an electric vaporizer;
Figures 3 and 4 are flow charts illustrating embodiments.

### Description of embodiments

The following embodiments are exemplary. Although the specification may refer to "an", "one", or "some" embodiment(s) in several locations, this does not necessarily mean that each such reference is to the same embodiment(s), or that the feature only applies to a single embodiment. Single features of different embodiments may also be combined to provide other embodiments.

Electric vaporizers are used for consuming or inhaling materials. Generally materials are in liquid which is heated by a heating element comprising a resistor. Electric power is fed to the heating element which vaporizes desired material for inhaling.

Figure 1 illustrates an example of an electric vaporizer 100. The vaporizer 100 of Figure 1 comprises a battery compartment 102 and a liquid compartment 104 comprising a heating element 106 and liquid 108 to be heated. Typically the liquid compartment 104 is detachable from the battery compartment 102. An electrical connection 110 connects the liquid compartment 104 and the battery compartment 102. The electric vaporizer 100 may comprise one or more buttons 112 and a display 114.

The user may change the liquid compartments 104 comprising a heating element 106. In addition, the heating element 106 of a liquid compartment 104 may be changed. The electrical properties of the heating elements may vary. For example if the heating element comprises a resistor wire, the electrical resistance of the resistor may vary. A typical value for the electrical resistance of a heating element varies between 0.3 to 10 ohms. The electrical resistance has an effect on the usage experience of the electronic vaporizer 100. There are devices on the market which let the user select a suitable power fed to the heating element when using the electronic vaporizer. However, these devices have some drawbacks. Typically the user is given a possibility to control the power over a given range. For example, the vaporizer may let the user to select a power between 5 to 15 watts. These solutions do not take the electrical properties of the heating element into account. Thus, it is possible for the user to select such a power which burns the heating element.

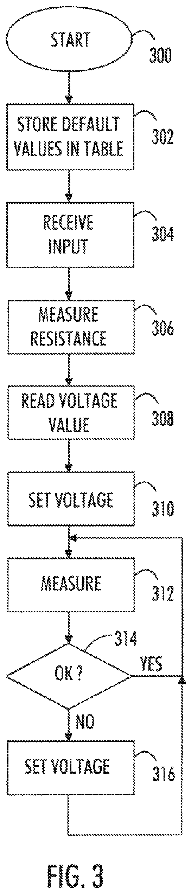

Let us study an example of the operation of the electric vaporizer in view of Figures 2 and 3. The embodiment starts at step 300. The electric vaporizer 100 comprises a controller 200 which controls the operation of the vaporizer. The vaporizer may comprise a memory 202 operationally connected to the controller 200. In some embodiments, the memory and the controller may be combined.

In step 302, the memory is configured to store a table comprising resistance values and a default power value for each resistance value. The power values may be predetermined on the basis of an experimental formula or empirical experiments, for example.

As illustrated in Figure 1, the electric vaporizer 100 comprises a liquid compartment 104 comprising a heating element 106. Typically the liquid compartment 104 is detachable. The heating element 106 comprises a resistor wire. In an embodiment, the electric vaporizer comprises a current sense amplifier 204 connected to the heating unit 104. The electric vaporizer comprises a battery 206 which provides the electrical power required by the apparatus. The electric vaporizer may further comprise a power controller unit 208 which may be configured to control the power fed from the battery 206 to the heating unit 104 and the current sense amplifier 204. The power controller unit 208 may operate under the control of the controller 200. In an embodiment, the power controller unit 208 is realized as a buck-boost controller. A buck-boost controller is configured to control the power fed to a load to be either smaller or greater than the power given by a source battery.

Regarding the reference numberings in Figure 1, the heating unit 104 is in practice the same as the liquid compartment. The heating unit 104 thus comprises the heating element 106 and liquid 108 to be heated and vaporized for the user to inhale.

The electric vaporizer 100 further comprises user interface 210 which may be realized with one or more buttons and a display, for example. In an embodiment, a button may be reserved for initializing vaporizing operation. In an embodiment, some other buttons may be used for controlling the vaporizing operation. The button may be realized with push buttons, touch pad or with any other technology available.

In step 304, the controller is configured to receive input from the user via the user interface 210. The input may be a command to initialize vaporizing operation.

In step 306, the controller is configured to measure the resistance of the heating unit 104. The measurement may be performed by giving a command to the power controller unit and the current sense amplifier. The controller 200 may configure the power controller 208 to output a given voltage to the heating unit. The current sense amplifier may measure the current (and also the voltage) and send measurements to the controller. The controller may calculate the resistance of the heating unit using formula R = U/I, where U is voltage and I current.

In step 308, the controller is configured to read from the memory 202 a default voltage value corresponding to the determined resistance.

The use of a default value enables the protection of the heating unit. The heating unit cannot be damaged accidentally. In known solutions, when a heating unit requiring a large voltage is changed to a unit requiring small voltage, the changed unit may accidentally receive too large voltage and get damaged. In addition, the use of tested default values provides immediately a satisfactory user experience to the user.

One advantage related to the structure of the present electronic vaporizer is that the use of a power controller enables the use of a large range of voltages or powers which can be fed to the heating unit.

In step 310, the controller is configured to give a command to the power controller unit 208 to feed the default voltage value to the heating unit.

In an embodiment, the controller may be configured to measure the power fed to the heating unit 104, compare the measured power to the determined power, and control the power source on the basis of the comparison. In step 312, the measurement is performed by the current sense amplifier. The controller may compare the measurement to the required value in step 314 and correct the voltage in step 316 if needed. This procedure may be executed at given intervals or a few times after the initialization of the vaporizing procedure.

The user manually adjusts the power fed to the heating unit using the user interface 210. For example, the user is given the possibility of adjusting the voltage fed into the heating unit in steps of 0.1 volts. Figure 4 illustrates this example. In step 400, the controller 200 detects that the user has indicated voltage up function. This is realized with a power up button, for example. In step 402, the controller instructs the power control unit to increase the voltage fed to the heating unit by 0.1 volts. There are some minimum and maximum values which the user is not allowed to exceed.

**Table 1**

| **Resistance** | **Min limit** | **Default** | **Max limit** |
|---|---|---|---|
| 0.5 | 1.9 | 2.3 | 2.7 |
| 0.6 | 2.0 | 2.6 | 3.0 |
| 0.7 | 2.4 | 3.0 | 3.5 |
| ... | ... | ... | ... |
| 5.4 | 5.9 | 7.4 | 8.7 |
| 5.5 | 6.2 | 7.8 | 9.0 |

Table 1 illustrates an example of data stored in the memory 202. The memory may store resistance values and corresponding default voltage or power values. In addition, for each resistance value a minimum and maximum value for the voltage or power may be stored.

The memory 202 may also store the current measured resistance value. If the same heating unit is used, the power or voltage values selected by the user may be used repeatedly. However, if the heating unit is changed such that the resistance changes, the controller 200 may detect the change and select the default power or voltage value for the determined resistance.

In an embodiment, the user is given the possibility to freely select any voltage or power value from a predetermined range, such as 2 to 8.2 volts, for example. In this mode, the resistance value measured from the heating unit has no effect.

The controller 200 may be implemented as an electronic digital computer, which may comprise a working memory (RAM), a central processing unit (CPU), and a system clock. The CPU may comprise a set of registers, an arithmetic logic unit, and a control unit. The control unit is controlled by a sequence of program instructions transferred to the CPU from the RAM. The control unit may contain a number of microinstructions for basic operations. The implementation of microinstructions may vary, depending on the CPU design. The program instructions may be coded by a programming language, which may be a high-level programming language, such as C, Java, etc., or a low-level programming language, such as a machine language, or an assembler. The electronic digital computer may also have an operating system, which may provide system services to a computer program written with the program instructions.

It will be obvious to a person skilled in the art that, as technology advances, the inventive concept can be implemented in various ways. The invention and its embodiments are not limited to the examples described above but may vary within the scope of the claims.

## Claims

1. A controller (200) of an electronic vaporizer (100), where the electronic vaporizer (100) comprises a heating unit (104), where the heating unit (104) comprises a heating element (106) and liquid to be heated, **characterized in that** the controller (200) is configured to:
store (302) a table comprising resistance values and a default power value for each resistance value; and when the heating unit (104) of the electronic vaporizer has been changed to a new heating unit (104), the controller (200) is configured to
measure (306) the resistance of the new heating unit (104) of the electronic vaporizer (100);
determine (308) a default power value for the measured resistance on the basis of the stored table for the new heating unit (104); and
control (310) a power source (206) to feed the new heating unit (104) with the determined default power,
wherein the determined default power value fed to the new heating unit (104) is further adjustable by a user to be below a given maximum power and above a given minimum power from the stored table for the measured resistance value.

2. The controller (200) of claim 1, **characterized in that** the controller (200) is further configured to detect (304) input of a user and measure (306) the resistance of the heating unit (104) after the detection.

3. The controller (200) of any preceding claim, **characterized in that** the controller (200) is further configured to measure (312) the power fed to the heating unit (104), compare (314) the measured power to the determined power, and control (316) the power source (206) on the basis of the comparison.

4. The controller (200) of any preceding claim, **characterized in that** the controller (200) is further configured to control (402) the power source (206) to decrease or increase the power fed to the heating unit (104) on the basis of input (400) from the user.

5. The controller (200) of any preceding claim, **characterized in that** the power fed to the heating unit (104) is limited to be below a given maximum power and above a given minimum power.

6. An electronic vaporizer (100), **characterized in that** the electronic vaporizer (100) comprises the controller (200) of any preceding claim 1-5.

7. A method for controlling an electronic vaporizer (100), where the electronic vaporizer (100) comprises a heating unit (104), where the heating unit (104) comprises a heating element (106) and liquid to be heated, **characterized in that** the method comprises the steps of:
storing (302) a table comprising resistance values and a default power value for each resistance value; and when the heating unit (104) of the electronic vaporizer has been changed to a new heating unit (104),
measuring (306) the resistance of the new heating unit (104) of the electronic vaporizer (100);
determining (308) a default power value for the measured resistance on the basis of the stored table for the new heating unit (104); and
controlling (310) a power source (206) to feed the new heating unit (104) with the determined default power
wherein the determined default power value fed to the new heating unit (104) is further adjustable by a user to be below a given maximum power and above a given minimum power from the stored table for the measured resistance value.

8. The method of claim 7, **characterized in that** it further comprises: detecting (304) input of a user and measuring (306) the resistance of the heating unit (104) after the detection.

9. The method of claim 7 or claim 8, **characterized in that** it further comprises: measuring (312) the power fed to the heating unit (104), comparing (314) the measured power to the determined power, and controlling (316) the power source (206) on the basis of the comparison.

## Patentansprüche

1. Ein Steuergerät (200) eines elektronischen Verdampfers (100), wobei der elektronische Verdampfer (100) eine Heizeinheit (104) umfasst, wobei die Heizeinheit (104) ein Heizelement (106) und zu erhitzende Flüssigkeit umfasst, **dadurch gekennzeichnet, dass** das Steuergerät (200) konfiguriert ist, um:
eine Tabelle zu speichern (302), die Widerstandswerte und einen Standardleistungswert für jeden Widerstandswert umfasst; und wenn die Heizeinheit (104) des elektronischen Verdampfers zu einer neuen Heizeinheit (104) gewechselt wurde, ist die Steuerung (200) konfiguriert, um
den Widerstand der neuen Heizeinheit (104) des elektronischen Verdampfers (100) zu messen (306);
einen Standardleistungswert für den gemessenen Widerstand auf der Grundlage der gespeicherten Tabelle für die neue Heizeinheit (104) zu bestimmen (308); und
eine Stromquelle (206) zu steuern (310), um die neue Heizeinheit (104) mit der bestimmten Standardleistung zu versorgen,
wobei der ermittelte Standardleistungswert, der der neuen Heizeinheit (104) zugeführt wird, ferner von einem Benutzer so eingestellt werden kann, dass er unter einer gegebenen maximalen Leistung und über einer gegebenen minimalen Leistung aus der gespeicherten Tabelle für den gemessenen Widerstandswert liegt.

2. Das Steuergerät (200) nach Anspruch 1, **dadurch gekennzeichnet, dass** das Steuergerät (200) ferner so konfiguriert ist, dass es die Eingabe eines Benutzers erkennt (304) und nach der Erkennung den Widerstand der Heizeinheit (104) misst (306).

3. Das Steuergerät (200) nach einem der vorhergehenden Ansprüche, **dadurch gekennzeichnet, dass** das Steuergerät (200) ferner so konfiguriert ist, dass es die der Heizeinheit (104) zugeführte Leistung misst (312), die gemessene Leistung mit der ermittelten Leistung vergleicht (314) und die Stromquelle (206) auf der Grundlage des Vergleichs steuert (316).

4. Das Steuergerät (200) nach einem der vorhergehenden Ansprüche, **dadurch gekennzeichnet, dass** das Steuergerät (200) ferner so konfiguriert ist, dass es die Energiequelle (206) steuert (402), um die der Heizeinheit (104) zugeführte Leistung auf der Grundlage einer Eingabe (400) des Benutzers zu verringern oder zu erhöhen.

5. Das Steuergerät (200) nach einem der vorhergehenden Ansprüche, **dadurch gekennzeichnet, dass** die der Heizeinheit (104) zugeführte Leistung so begrenzt ist, dass sie unter einer gegebenen maximalen Leistung und über einer gegebenen minimalen Leistung liegt.

6. Ein elektronischer Verdampfer (100), **dadurch gekennzeichnet, dass** der elektronische Verdampfer (100) das Steuergerät (200) nach einem der vorhergehenden Ansprüche 1-5 umfasst.

7. Ein Verfahren zum Steuern eines elektronischen Verdampfers (100), wobei der elektronische Verdampfer (100) eine Heizeinheit (104) umfasst, wobei die Heizeinheit (104) ein Heizelement (106) und eine zu erhitzende Flüssigkeit umfasst, **dadurch gekennzeichnet, dass** das Verfahren die folgenden Schritte umfasst:
das Speichern einer Tabelle (302), die Widerstandswerte und einen Standardleistungswert für jeden Widerstandswert umfasst; und wenn die Heizeinheit (104) des elektronischen Verdampfers zu einer neuen Heizeinheit (104) gewechselt wurde,
das Messen (306) des Widerstands der neuen Heizeinheit (104) des elektronischen Verdampfers (100);
das Bestimmen (308) eines Standardleistungswerts für den gemessenen Widerstand auf der Grundlage der gespeicherten Tabelle für die neue Heizeinheit (104); und
das Steuern (310) einer Stromquelle (206), um die neue Heizeinheit (104) mit der bestimmten Standardleistung zu versorgen,
wobei der ermittelte Standardleistungswert, der der neuen Heizeinheit (104) zugeführt wird, ferner von einem Benutzer so eingestellt werden kann, dass er unter einer gegebenen maximalen Leistung und über einer gegebenen minimalen Leistung aus der gespeicherten Tabelle für den gemessenen Widerstandswert liegt.

8. Das Verfahren nach Anspruch 7, **dadurch gekennzeichnet, dass** es ferner Folgendes umfasst: Erkennung (304) der Eingabe eines Benutzers und Messen (306) des Widerstands der Heizeinheit (104) nach der Erkennung.

9. Das Verfahren nach Anspruch 7 oder Anspruch 8, **dadurch gekennzeichnet, dass** es ferner Folgendes umfasst: Messen (312) der der Heizeinheit (104) zugeführten Leistung, Vergleichen (314) der gemessenen Leistung mit der ermittelten Leistung und Steuern (316) der Stromquelle (206) auf der Grundlage des Vergleichs.

## Revendications

1. Dispositif de commande (200) d'un vaporisateur électronique (100), où le vaporisateur électronique (100) comprend une unité de chauffage (104), où l'unité de chauffage (104) comprend un élément de chauffage (106) et un liquide à chauffer, **caractérisé en ce que** le dispositif de commande (200) est configuré pour :
stocker (302) un tableau comprenant des valeurs de résistance et une valeur de puissance par défaut pour chaque valeur de résistance ; et lorsque l'unité de chauffage (104) du vaporisateur électronique a été remplacée par une nouvelle unité de chauffage (104), le dispositif de commande (200) est configuré pour
mesurer (306) la résistance de la nouvelle unité de chauffage (104) du vaporisateur électronique (100) ;
déterminer (308) une valeur de puissance par défaut pour la résistance mesurée sur la base du tableau stocké pour la nouvelle unité de chauffage (104) ; et
commander (310) une source d'énergie (206) pour alimenter la nouvelle unité de chauffage (104) avec la puissance par défaut déterminée,
dans lequel la valeur de puissance par défaut déterminée fournie à la nouvelle unité de chauffage (104) est en outre réglable par un utilisateur pour être inférieure à une puissance maximale donnée et supérieure à une puissance minimale donnée à partir du tableau stocké pour la valeur de résistance mesurée.

2. Dispositif de commande (200) selon la revendication 1, **caractérisé en ce que** le dispositif de commande (200) est en outre configuré pour détecter (304) l'entrée d'un utilisateur et mesurer (306) la résistance de l'unité de chauffage (104) après la détection.

3. Dispositif de commande (200) selon une quelconque revendication précédente, **caractérisé en ce que** le dispositif de commande (200) est en outre configuré pour mesurer (312) la puissance fournie à l'unité de chauffage (104), comparer (314) la puissance mesurée à la puissance déterminée, et commander (316) la source d'énergie (206) sur la base de la comparaison.

4. Dispositif de commande (200) selon une quelconque revendication précédente, **caractérisé en ce que** le dispositif de commande (200) est en outre configuré pour commander (402) la source d'énergie (206) afin de diminuer ou d'augmenter la puissance fournie à l'unité de chauffage (104) sur la base d'une entrée (400) de l'utilisateur.

5. Dispositif de commande (200) selon une quelconque revendication précédente, **caractérisé en ce que** la puissance fournie à l'unité de chauffage (104) est limitée pour être inférieure à une puissance maximale donnée et supérieure à une puissance minimale donnée.

6. Vaporisateur électronique (100), **caractérisé en ce que** le vaporisateur électronique (100) comprend le dispositif de commande (200) selon une quelconque revendication précédente 1 à 5.

7. Procédé de commande d'un vaporisateur électronique (100), où le vaporisateur électronique (100) comprend une unité de chauffage (104), où l'unité de chauffage (104) comprend un élément de chauffage (106) et un liquide à chauffer, **caractérisé en ce que** le procédé comprend les étapes suivantes :
le stockage (302) d'un tableau comprenant des valeurs de résistance et une valeur de puissance par défaut pour chaque valeur de résistance ; et lorsque l'unité de chauffage (104) du vaporisateur électronique a été remplacée par une nouvelle unité de chauffage (104),
la mesure (306) de la résistance de la nouvelle unité de chauffage (104) du vaporisateur électronique (100) ;
la détermination (308) d'une valeur de puissance par défaut pour la résistance mesurée sur la base du tableau stocké pour la nouvelle unité de chauffage (104) ; et
la commande (310) d'une source d'énergie (206) pour alimenter la nouvelle unité de chauffage (104) avec la puissance par défaut déterminée,
dans lequel la valeur de puissance par défaut déterminée fournie à la nouvelle unité de chauffage (104) est en outre réglable par un utilisateur pour être inférieure à une puissance maximale donnée et supérieure à une puissance minimale donnée à partir du tableau stocké pour la valeur de résistance mesurée.

8. Procédé selon la revendication 7, **caractérisé en ce qu'**il comprend en outre : la détection (304) de l'entrée d'un utilisateur et la mesure (306) de la résistance de l'unité de chauffage (104) après la détection.

9. Procédé selon la revendication 7 ou 8, **caractérisé en ce qu'**il comprend en coutre : la mesure (312) de la puissance fournie à l'unité de chauffage (104), la comparaison (314) de la puissance mesurée à la puissance déterminée, et la commande (316) de la source d'énergie (206) sur la base de la comparaison.
